# EUROPÄISCHE PATENTSCHRIFT

(11) **EP 0 900 778 B1**
(45) Veröffentlichungstag und Bekanntmachung des Hinweises auf die Patenterteilung: **28.11.2001**
(21) Anmeldenummer: 98115207.7
(22) Anmeldetag: 13.08.1998
(51) Int. Cl.: C07C 67/60, C07C 69/54

(54) **Verfahren zur Herstellung von Estern ethylenisch ungesättigter Carbonsäuren und deren Verwendung**
Process for the preparation of esters of ethylenically unsaturated carboxylic acids and their use
Procédé de préparation d'esters d'acides carboxyliques à insaturation éthylénique et leur utilisation

(30) Priorität: 26.08.1997 DE 19737017
(43) Veröffentlichungstag der Anmeldung: 10.03.1999
(73) Patentinhaber: BAYER AG, 51368 Leverkusen (DE)
(72) Erfinder: Fischer, Wolgang, Dr., 40668 Meerbusch (DE); Margotte, Dieter, Dr., 47807 Krefeld (DE); Meixner, Jürgen, Dr., 47803 Krefeld (DE)

(56) Entgegenhaltungen:
- EP-A- 0 054 105
- EP-A- 0 279 303

## Beschreibung

Die Erfindung betrifft ein Verfahren zur Herstellung von Estern aus ein- oder mehrwertigen Alkoholen bzw. Estervorstufen mit mindestens zwei Hydroxylgruppen pro Molekül, hergestellt aus mehrwertigen Alkoholen und ein- oder zweiwertigen, gesättigten oder aromatisch ungesättigten Carbonsäuren und ethylenisch ungesättigten Carbonsäuren wie (Meth)Acrylsäure durch säurekatalysierte Azeotropveresterung, wobei die Katalysatorsäure sowie während der Veresterung nicht umgesetzte Carbonsäuregruppen durch anschließende Reaktion mit ethylenisch ungesättigten Monoepoxiden umgesetzt werden.

Ester der (Meth)Acrylsäure finden in der Beschichtungstechnologie sowie in Spachtelmassen Anwendung z.B. als Reaktivverdünner. Hierunter sind (Meth)Acrylsäureester auf Basis von ein- oder mehrwertigen Alkoholen zu verstehen. Die Herstellung dieser (Meth)Acrylsäureester erfolgt im allgemeinen durch azeotrope Veresterung von (Meth)Acrylsäure mit ein- oder mehrwertigen Alkoholen in einem inerten Lösemittel unter Verwendung saurer Katalysatoren, in Gegenwart von Stabilisatoren und unter Durchleiten von Luft. Nach der Veresterung wird das inerte Lösemittel abdestilliert. Eingesetzt werden Reaktivverdünner in Kombination mit ungesättigten, gelegentlich auch mit gesättigten Lackharzen zur Einstellung applikationsgerechter Viskositäten. Die Härtung entsprechender Zubereitungen kann erfolgen durch Peroxid/Beschleunigersysteme (konventionelle Härtung), durch UV-Strahlung unter Verwendung von Photoinitiatoren oder direkt unter Elektronenstrahlung.

Weiterhin werden sie in Form von Polyetheracrylaten eingesetzt. Diese Verbindungsklasse wird analog den Reaktivverdünnern durch Azeotropveresterung von (Meth)Acrylsäure mit höherfunktionellen Polyetheralkoholen hergestellt. Einsatzbereich sind hauptsächlich UV- oder EB-härtende Beschichtungszubereitungen. Polyetheracrylate können mit ungesättigten Lackharzen, Reaktivverdunnern oder inerten Lösemitteln kombiniert werden.

Ein weiteres Anwendungsgebiet ist die Verwendung in Form von Polyesteracrylatharzen. Zur Herstellung der sog. Polyesteracrylatharze werden Prekondensate aus mehrwertigen Alkoholen und ein- oder zweiwertigen, gesättigten oder aromatisch ungesättigten Carbonsäuren, durch Schmelzkondensation erzeugt. Diese Prekondensate müssen mindestens zwei Hydroxylgruppen pro Molekül aufweisen und werden dann analog der Reaktivverdünner oder der Polyetheracrylate durch sauer katalysierte, azeotrope Veresterung mit (Meth)Acrylsäure umgesetzt. Die Polyesteracrylate werden ebenfalls überwiegend durch energierreiche Strahlung gehärtet und können in Kombination mit inerten Lösemitteln oder Reaktiwerdünnern zur Anwendung kommen.

Die genannten (Meth)Acrylsäureester können auch als Vorstufen zur Herstellung für andere ungesättigte Harze wie z.B. Urethanacrylate oder aminmodifizierte Polyetheracrylate eingesetzt werden.

Bei der Herstellung der (Meth)Acrylsäureester nach den obengenannten bekannten Verfahren bleibt meist eine Restsäurezahl von bis zu 20 (mg KOH/g Substanz) bestehen. Diese Restsäurezahl verleiht den Produkten jedoch korrosive Eigenschaften, die z.B. zur Rostbildung in Behältern führen kann, mit der Konsequenz der Kontamination des Produktes und dessen Destabilisation durch Metallionen. Reste an (Meth)Acrylsäure können sich auch durch Geruch und evtl. Hautreizune bei der Handhabung bemerkbar machen. Daher gab es vor allem zur Entfernung der Restsäuren aus den hergestellten Harzen viele Vorschläge.

So wird bei der Herstellung der (Meth)Acrylsäureester nach der Azeotropveresterung mit Wasser, gegebenenfalls verdünnter Lauge gewaschen. Dadurch werden die Katalysatorsäure und gegebenenfalls weitere Säuren entfernt. Es bleibt jedoch meist eine Restsäurezahl von bis zu 5 (mg KOH/g Substanz) bestehen. Desweiteren können sich schwer trennbare Emulsionen bilden.

Man hat versucht, das Problem auch dadurch zu lösen, daß nach der Herstellung des (Meth)Acrylsäureesters gelöschter Kalk zugegeben und das entstehende unlösliche Kalziumsalz durch Filtration entfernt wird (z.B. US-A-3.717.672). Hierbei bilden sich jedoch oft schmierige und nur schwer filtrierbare Niederschläge.

Weiterhin sind Verfahren zur Herstellung von (Meth)Acrylsäureestern bekannt, bei denen nach der Neutralisation des Veresterungskatalysators die restliche (Meth)Acrylsäure mit einer Epoxyverbindung umgesetzt wird (z.B. EP-A 127 766, EP-A 54 105). Je nach Art und Menge des eingesetzten Epoxids ändert sich dabei zusätzlich die Viskosität des Endproduktes in unterschiedlichem Ausmaß. Darüber hinaus bleiben die Neutralisationsprodukte als nicht polymerisationsfähige, d.h. als nicht einbaubare Bestandteile im Bindemittel, was bei späterer Härtung zu unerwünschten Filmeigenschaften, z.B. niedrige Härte oder Extrahierbarkeit führen kann.

Es war daher wünschenswert, (Meth)Acrylsäureester so herzustellen, daß das Endprodukt eine verringerte Säurezahl besitzt, ohne daß die Eigenschaften der damit hergestellten polymerisationsfähigen Gemische, wie Farbzahl, Viskosität oder Reaktivität, sowie die Eigenschaften der daraus hergestellten Lackfilme, wie Härte oder Extrahierbarkeit nachteilig beeinflußt werden.

Die Aufgabe wurde dadurch gelöst, daß die eingesetzte Katalysatorsäure, sowie die restliche (Meth)Acrylsäure nach Herstellung des gewünschten (Meth)Acrylsäureesters mit einer ethylenisch ungesättigten Monoepoxyverbindung umgesetzt wird. Hierdurch wird eine deutliche Verringerung der Säurezahl auf Werte von unter 1 (mg KOH/g Substanz) erzielt und das resultierende Bindemittel enthält ausschließlich einbaubare, polymerisationsfähige Reaktionsprodukte.

Gegenstand der Erfindung ist demnach ein Verfahren zur Herstellung von Estern aus ein- oder mehrwertigen Alkoholen bzw. Estervorstufen mit mindestens zwei Hydroxylgruppen pro Molekül, hergestellt aus mehrwertigen Alkoholen und ein- oder zweiwertigen Carbonsäuren, sowie gegebenenfalls auch die entsprechenden Anhydride, und ethylenisch ungesättigten Carbonsäuren wie (Meth)Acrylsäure durch säurekatalysierte Azeotropveresterung, in Gegenwart von 0.01 bis 1 Gew.-%, bezogen auf das zu veresternde Gemisch aus (Meth)Acrylsäure und Alkohol, eines oder mehrerer Polymerisationsinhibitoren, dadurch gekennzeichnet, daß die Katalysatorsäure, sowie während der Veresterung nicht umgesetzte Carbonsäuregruppen, durch anschließende Reaktion mit ethylenisch ungesättigten Monoepoxyverbindungen umgesetzt werden.

Als ethylenisch ungesättigte Säure im erfindungsgemäßen Verfahren kann sowohl Acrylsäure als auch Methacrylsäure sowie ein Gemisch beider eingesetzt werden.

Als Alkohole für das erfindungsgemäße Verfahren können ein- oder mehrwertige, gesättigte, aliphatische oder cycloaliphatische Alkohole eingesetzt werden, die gegebenenfalls Ethergruppen enthalten. Solche Alkohole haben Molekulargewichte von 32 bis etwa 800. Beispielhaft seien folgende Alkohole genannt: Methanol, Ethanol, Propanole, Butanole, Hexanole, Cetylalkohol, Stearylalkohol, Ethylenglykol, Propylenglykol, Butandiol-1,4, Pentandiol-1,5, Neopentylglykol, Hexandiol-1,6, Glycerin, Trimethylolethan, Trimethylolpropan, Pentaerythrit, 2-Ethylhexanol, Cyclohexanol, Dimethylolcyclohexan sowie Oxalkylierungsprodukte der genannten Alkohole mit beispielsweise 1 bis 5 Mol Ethylenoxid oder Propylenoxid pro Hydroxyl-Äquivalent. Die zur Herstellung der Estervorstufen geeigneten Alkohole sind die obengenannten mehrwertigen Alkohole, die geeigneten, gesättigten oder aromatisch ungesättigten Carbonsäuren können sein: Monocarbonsäuren, wie z.B. Bernsteinsäure, Glutarsäure, Adipinsäure, Phthal-, Isophthal- und Terephthalsäure, substituierte Phthalsäuren, gegebenenfalls auch die entsprechenden Säureanhydride.

Als saure Veresterungskatalysatoren werden anorganische oder organische Säuren in einer Menge von 0,1 bis 3 Gew.-%, bezogen auf das Gewicht der zu veresternden Reaktionskomponenten, eingesetzt. Beispiele für solche Veresterungskatalysatoren sind Schwefelsäure, Phosphorsäure, Pyrophosphorsäure, p-Toluolsulfonsäure, Styroldivinylbenzol-sulfonsäure, Chlorsulfonsäure, Chlorameisensäure, bevorzugt Schwefelsäure und p-Toluolsulfonsäure.

Das erfindungsgemäße Verfahren wird in einem Lösemittel durchgeführt, das mit Wasser nicht mischbar ist und mit Wasser im Sinne einer Wasserdampf-Destillation destillierbar ist. Hierfür kommen Kohlenwasserstoffe sowie deren Halogen- oder Nitro-Substitutionsprodukte in Betracht sowie weitere Lösungsmittel, die weder mit den Reaktionspartnern reagieren noch sich unter dem Einfluß der sauren Katalysatoren verändern.

In bevorzugter Weise werden nicht substituierte Kohlenwasserstoffe eingesetzt. Beispielhaft seien genannt: aliphatische Kohlenwasserstoffe, wie Hexan, Heptan, Octan, Benzinfraktionen verschiedener Siedebereiche, cycloaliphatische Kohlenwasserstoffe, wie Cyclopentan, Cyclohexan, Methyl-cyclohexan, oder aromatische Kohlenwasserstoffe, wie Benzol, Toluol oder die isomeren Xylole. In bevorzugter Weise werden solche Lösemittel eingesetzt, die im Bereich von 70 bis 120°C sieden. Insbesondere seien hier Cyclohexan, Toluol oder Benzinfraktionen im Siedebereich von 70 bis 120°C genannt. Das mit Wasser nicht mischbare Lösemittel kann auch ein Gemisch der obengenannten Stoffe sein. Es wird in einer Menge von 10 bis 100 Gew.-%, bevorzugt 15 bis 50 Gew.-%, besonders bevorzugt 20 bis 40 Gew.-%, bezogen auf das Gewicht der zu veresternden Reaktionskomponenten, eingesetzt.

Zur Herstellung von Polyesteracrylaten wird dem Reaktionsschritt im Lösemittel eine lösemittelfreie Schmelzkondensation der beschriebenen Alkoholkomponenten mit den beschriebenen gesättigten bzw. ungesättigten Carbonsäuren vorangestellt und dieses so erhaltene Prekondensat mit (Meth)Acrylsäure weiter in einem der beschriebenen Lösemittel azeotrop verestert.

Das erfindungsgemäße Verfahren wird in Gegenwart eines oder mehrerer Polymerisationsinhibitoren in einer Menge von 0,01 bis 1 Gew.-%, bevorzugt 0,1 bis 0,5 Gew.-%, bezogen auf das zu veresternde Gemisch aus (Meth)Acrylsäure und Alkohol, durchgeführt.

Solche Inhibitoren sind beispielsweise in Houben-Weyl, Methoden der organischen Chemie, 4. Auflage, Band XIV/1, Georg Thieme Verlag, Stuttgart 1961, Seite 433 ff., beschrieben. Als Beispiele seien genannt: Natriumdithionit, Natriumhydrogensulfid. Schwefel, Hydrazin, Phenylhydrazin, Hydrazobenzol, N-Phenyl-ß-naphthylamin, N-Phenyl-ethanolamin, Dinitrobenzol, Picrinsäure, p-Nitrosodimethylanilin. Diphenylnitrosamin, Phenole, wie p-tert.-Butyl-brenzcatechin, 2,5-Di-tert.-amylhydrochinon, p-Alkoxyphenole, Di-tert.-butylhydrochinon, Tetramethyl-thiuramdisulfid, 2-Mercaptobenzthiazol, Dimethyl-dithiocarbaminsäure-natriumsalz usw.

Weiterhin wird in einer bevorzugten Variante des erfindungsgemäßen Verfahrens ein sauerstoffhaltiges Gas, vorzugsweise Luft bzw. Mischungen aus Sauerstoff und inerten Gasen in das lösemittelhaltige Reaktionsgemisch eingeleitet.

Entsprechend dem erfindungsgemäßen Verfahren wird zunächst die Veresterung der (Meth)Acrylsäure in einem Temperaturbereich von 60 bis 140°C, bevorzugt 70 bis 120°C, besonders bevorzugt beim Siedepunkt des eingesetzten Lösemittels durchgeführt. Hierbei wird ständig Lösemittel aus dem Reaktionsgemisch destillativ abgezogen, außerhalb des Reaktionsgefäßes in einem Wasserabscheider von herausgeschlepptem Wasser abgetrennt und danach wieder in das Reaktionsgemisch zurückgeführt. Das Ende der Reaktion ist erreicht, wenn kein weiteres Reaktionswasser mehr aus dem Reaktionsgefäß herausgeschleppt wird.

Nach beendeter Veresterung erfolgt die Umsetzung mit der ethylenisch ungesättigten Epoxyverbindung. In bevorzugter Weise wird diese nach der Abdestillation des mit Wasser nicht mischbaren Lösemittels durchgeführt.

Beispiele für erfindungsgemäß einsetzbare ethylenisch ungesättigte mono-Epoxyverbindungen sind: Glycidylacrylat (2,3-Epoxypropylacrylat), Glycidylmethacrylat (2,3-Epoxypropylmethacrylat), sowie Additionsprodukte aus 1 mol Acrylsäure oder Methacrylsäure und Bisepoxyverbindungen wie z.B. Hexandiolbisglycidylether, Bisphenol-A-bis-glycidylether, Hexahydrophthalsäurebisglycidylester. Der Einsatz der erfindungsgemäßen Epoxyverbindung erfolgt im Verhältnis von 2 Val Epoxygruppen zu 1 Val Säuregruppen bis zu 1,2 Val Epoxygruppen zu 1 Val Säuregruppen, bevorzugt im Verhältnis von 1,6 Val Epoxygruppen zu 1 Val Säuregruppen. Die Umsetzung erfolgt bei erhöhter Temperatur, im allgemeinen bei 80 bis 120°C, vorzugsweise 90 bis 110°C.

Gegebenenfalls kann die Umsetzung der ethylenisch ungesättigten Monoepoxyverbindung mit Säure in Gegenwart von Katalysatoren erfolgen. Geeignete Katalysatoren sind: Quartäre Ammoniumhalogenide wie z.B. Tetrabutylammoniumbromid, -iodid; Triphenylphosphin; Phosphoniumsalze wie z.B. Ethyltriphenylphosphoniumiodid, Alkalihalogenid wie z. B. Kaliumiodid

Die Umsetzung erfolgt so lange, bis die Säurezahl auf den Wert von <1 (mg KOH/g Substanz) gefallen ist.

Die erfindungsgemäß erhältlichen (Meth)Acrylsäureester finden Verwendung als Bindemittel oder Reaktivverdünner in strahlenhärtenden oder konventionell härtenden Spachtel- und Überzugsmassen. Die Erfindung betrifft daher auch die Verwendung der in beschriebenen Verfahren erhältlichen (Meth)Acrylsäureester.

### Beispiele

### Beispiel 1

### Herstellung eines Acrylsäureesters auf Basis eines Etherpolyols

310 g eines Triols, hergestellt aus Trimethylolpropan und 4 Mol Ethylenoxid und 187,2 g Acrylsäure werden zusammen mit 1,5 Gew.-% (bezogen auf die Summe aus Polyol und Acrylsäure) p-Toluolsulfonsäure, sowie mit 3000 ppm p-Methoxyphenol und 200 ppm 2,5-Di-tert-butylhydrochinon eingewogen und unter Rühren mit 160 g Isooctan gemischt. Unter Durchleitung von Luft (einfaches Kesselvolumen pro Stunde) und Überleitung von Stickstoff (doppeltes Kesselvolumen pro Stunde) wird unter andauerndem Rühren auf Rückflußtemperatur aufgeheizt (ca. 100°C). Das entstehende Reaktionswasser wird abgeschieden und der Rückfluß so lange aufrechterhalten bis eine Säurezahl von ca. 5 (mg KOH/g Substanz) erreicht ist. Danach wird auf 50°C abgekühlt und unter Vakuum Isooctan abdestilliert, bis bei 90°C und 50 mbar Druck nichts mehr übergeht. Die Apparatur wird belüftet und auf 60°C gekühlt, bei 60°C werden 8,52 g Glycidylmethacrylat zugegeben, unter Durchleiten von Luft und Überleiten von Stickstoff (Bedingungen siehe oben) auf 100°C erhitzt und 1 Stunde bei 100°C gehalten. Danach wird abgekühlt. Der entstandene Acrylsäureester ist klar, hat eine Säurezahl von <1 (mg KOH/g Substanz) und eine Viskosität von 150 bis 200 mPa.s bei 23°C.

### Beispiel 2

### Herstellung eines Polyesteracrylates

195,2 g Bernsteinsäureanhydrid, 215,9 g Terephthalsäure, 174,3 g Trimethylolpropan, 413,8 g Diethylenglykol und 99,5 g eines Triols, hergestellt aus Trimethylolpropan und 4 Mol Ethylenoxid, werden eingewogen und unter Überleitung von Stickstoff (doppeltes Kesselvolumen pro Stunde) aufgeheizt, ab 80°C unter Rühren. Von 160°C an wird so auf 230°C erhitzt, daß der austretende Gas/Wasserstrom 105°C (Kopftemperatur) nicht übersteigt. 230°C werden so lange gehalten, bis eine Säurezahl von <3 (mg KOH/g Substanz) erreicht ist (ca. 5 Stunden). Danach wird abgekühlt auf 40°C und die Apparatur zur azeotropen Veresterung (Wasserabscheider) umgebaut. Unter Durchleitung von Luft (einfaches Kesselvolumen pro Stunde) und Überleitung von Stickstoff (doppeltes Kesselvolumen pro Stunde) werden zu der hergestellten Polyestervorstufe 240,3 g Cyclohexan, 351 g Acrylsäure und -jeweils bezogen auf die Summe aus Polyestervorstufe und Acrylsäure - 1,5% p-Toluolsulfonsäure, 3000 ppm p-Methoxyphenol und 200 ppm Di-tert.-butylhydrochinon zugegeben. Unter Rühren wird auf Rückfluß erhitzt (ca. 85 bis 90°C) und unter starkem Rückfluß gehalten bis eine Säurezahl von <4 (mg KOH/g Substanz) erreicht ist. Die Apparatur wird abgekühlt auf 40°C und zur Destillation umgebaut. Es wird ein Vakuum von ca. 50 mbar angelegt und Cyclohexan so lange abdestilliert, bis bei 50 mbar und 90°C nichts mehr übergeht. Bei 60°C werden 27,7 g Glycidylmethacrylat zugegeben und unter Durchleiten von Luft und Überleiten von Stickstoff auf 100°C erhitzt und 2 Stunden bei dieser Temperatur gehalten. Danach wird abgekühlt. Das entstandene Polyesteracrylat ist klar, hat eine Säurezahl von <1 (mg KOH/g Substanz) und eine Viskosität von ca. 7000 mPa.s bei 23°C.

## Patentansprüche

1. Verfahren zur Herstellung von Estern aus ein- oder mehrwertigen Alkoholen oder Estervorstufen mit mindestens zwei Hydroxylgruppen pro Molekül, hergestellt aus mehrwertigen Alkoholen und ein oder zweiwertigen, gesättigten oder aromatisch ungesättigten Dicarbonsäuren, sowie gegebenenfalls auch die entsprechenden Anhydride, und ethylenisch ungesättigten Carbonsäuren durch säurekatalysierte Azeotropveresterung in Gegenwart von 0.01 bis 1 Gew.-%, bezogen auf das zu veresternde Gemisch aus (Meth)Acrylsäure und Alkohol, eines oder mehrerer Polymerisationsinhibitoren, **dadurch gekennzeichnet, daß** die Katalysatorsäure sowie nicht umgesetzte Carbonsäuregruppen durch anschließende Reaktion mit ethylenisch ungesättigten Monoepoxiden umgesetzt werden.

2. Verfahren nach Anspruch 1, **dadurch gekennzeichnet, daß** die ethylenisch ungesättigten Carbonsäuren, Acrylsäure oder Methacrylsäure oder ein Gemisch der beiden ist.

3. Verfahren nach Anspruch 1, **dadurch gekennzeichnet, daß** das ethylenisch ungesättigte Monoepoxid, Glycidylacrylat oder Glycidylmethacrylat oder ein Gemisch der beiden ist.

4. Verfahren nach Anspruch 1, **dadurch gekennzeichnet, daß** das ethylenisch ungesättigte Monoepoxid ein Reaktionsprodukt aus 1 Mol Bisepoxid und 1 Mol Acrylsäure oder Methacrylsäure ist.

5. Verfahren nach Anspruch 1, **dadurch gekennzeichnet, daß** 1,2 bis 2,0 Val Epoxid pro Mol Äquivalent Restsäure eingesetzt wird.

6. Verfahren nach Anspruch 1 bis 6, **dadurch gekennzeichnet, daß** die Umsetzung von Restsäure mit Epoxid bei 80 bis 120°C durchgeführt wird.

## Claims

1. A process for the preparation of esters from monohydric or polyhydric alcohols or from ester precursors having at least two hydroxyl groups per molecule, prepared from polyhydric alcohols and monobasic or dibasic, saturated or aromatically unsaturated dicarboxylic acids and optionally the corresponding anhydrides, and ethylenically unsaturated carboxylic acids by acid-catalysed azeotropic esterification, in the presence of 0.01 to 1 wt.% of one or more polymerisation inhibitors, based on the mixture of (meth)acrylic acid and alcohol to be esterified, **characterised in that** the acid catalyst as well as unreacted carboxylic acid groups are reacted by subsequent reaction with ethylenically unsaturated monoepoxides.

2. A process according to claim 1, **characterised in that** the ethylenically unsaturated carboxylic acids are acrylic acid or methacrylic acid or a mixture of the two.

3. A process according to claim 1, **characterised in that** the ethylenically unsaturated monoepoxide is glycidyl acrylate or glycidyl methacrylate or a mixture of the two.

4. A process according to claim 1, **characterised in that** the ethylenically unsaturated monoepoxide is a reaction product of 1 mol bisepoxide and 1 mol acrylic acid or methacrylic acid.

5. A process according to claim 1, **characterised in that** 1.2 to 2.0 g. equiv. epoxide is used per mol equivalent residual acid.

6. A process according to claim 1, **characterised in that** the reaction of residual acid with epoxide is carried out at 80°C to 120°C.

## Revendications

1. Procédé pour la préparation d'esters à partir d'alcools monovalents ou polyvalents ou de précurseurs d'esters contenant au moins deux groupes hydroxyle par molécule, préparés à partir d'alcools polyvalents et d'acides dicarboxyliques monovalents ou bivalents, saturés ou aromatiques insaturés, ainsi que le cas échéant des anhydrides correspondants, et d'acides carboxyliques à insaturation éthylénique par estérification azéotrope catalysée par un acide en présence d'un ou de plusieurs inhibiteurs de la polymérisation à concurrence de 0,01 à 1 % en poids rapporté au mélange à estérifier constitué de l'acide (méth)acrylique et de l'alcool, **caractérisé en ce qu'**on fait réagir l'acide faisant office de catalyseur ainsi que des groupes d'acides carboxyliques n'ayant pas réagi par réaction ultérieure avec des monoépoxydes à insaturation éthylénique.

2. Procédé selon la revendication 1, **caractérisé en ce que** les acides carboxyliques à insaturation éthylénique sont l'acide acrylique ou l'acide méthacrylique ou un mélange des deux.

3. Procédé selon la revendication 1, **caractérisé en ce que** le monoépoxyde à insaturation éthylénique est l'acrylate de glycidyle ou le méthacrylate de glycidyle ou un mélange des deux.

4. Procédé selon la revendication 1, **caractérisé en ce que** le monoépoxyde à insaturation éthylénique est un produit réactionnel de 1 mole de bisépoxyde et de 1 mole d'acide acrylique ou d'acide méthacrylique.

5. Procédé selon la revendication 1, **caractérisé en ce qu'**on met en oeuvre de 1,2 à 2,0 éq. d'époxyde par équivalent molaire d'acide résiduel.

6. Procédé selon les revendications 1 à 5, **caractérisé en ce qu'**on effectue la mise en réaction de l'acide résiduel avec l'époxyde à une température de 80 à 120°C.
